# EUROPÄISCHE PATENTSCHRIFT

(11) **EP 0 804 466 B1**
(45) Veröffentlichungstag und Bekanntmachung des Hinweises auf die Patenterteilung: **02.12.1998**
(21) Anmeldenummer: 95917962.3
(22) Anmeldetag: 26.04.1995
(51) Int. Cl.: C07K 5/10

(54) **NEUE TETRAPEPTIDE, IHRE HERSTELLUNG UND VERWENDUNG**
NOVEL TETRAPEPTIDES, THEIR MANUFACTURE AND USE
NOUVEAUX TETRAPEPTIDES, LEUR PRODUCTION ET LEUR UTILISATION

(30) Priorität: 06.05.1994 DE 4415998
(43) Veröffentlichungstag der Anmeldung: 05.11.1997
(73) Patentinhaber: BASF AKTIENGESELLSCHAFT, 67056 Ludwigshafen (DE)
(72) Erfinder: AMBERG, Wilhelm, D-61381 Friedrichsdorf (DE); BERNARD, Harald, D-67098 Bad Dürkheim (DE); BUSCHMANN, Ernst, D-67069 Ludwigshafen (DE); HAUPT, Andreas, D-68723 Schwetzingen (DE); JANITSCHKE, Lothar, D-67259 Kleinniedesheim (DE); JANSSEN, Bernd, D-67063 Ludwigshafen (DE); KARL, Ulrich, D-67061 Ludwigshafen (DE); KLING, Andreas, D-68259 Mannheim (DE); MÜLLER, Stefan, D-67346 Speyer (DE); DE POTZOLLI, Bernd, D-67098 Bad Dürkheim (DE); RITTER, Kurt, D-69115 Heidelberg (DE); THYES, Marco, D-67061 Ludwigshafen (DE); ZIERKE, Thomas, D-67459 Böhl-Iggelheim (DE)
(86) Internationale Anmeldenummer: EP9501576
(87) Internationale Veröffentlichungsnummer: WO9530690

(56) Entgegenhaltungen:
- JOURNAL OF THE AMERICAN CHEMICAL SOCIETY., Bd.113, Nr.17, 14. August 1991, GASTON, PA US Seiten 6692 - 6693 G R PETTITT ET AL. 'Antineoplastic agents 220. Synthesis of natural (-) dolastatin 15' in der Anmeldung erwähnt
- J JONES 'The chemical synthesis of peptides' 1991 , CLARENDON PRESS, OXFORD in der Anmeldung erwähnt see pages 47-51
- COLLECTION OF CZECHOSLOVAK CHEMICAL COMMUNICATIONS., Bd.27, 1962, PRAGUE CS Seiten 1273 - 1277 M ZAORAL ET AL. 'Amino acids and peptides. XXXVI. Pivaloyl chloride as a reagent in the mixed anhydride synthesis of peptides'

## Beschreibung

Gegenstand der Erfindung sind neue Tetrapeptide, ihre Herstellung und Verwendung.

Dolastatin 15 ist eine in der US-PS 4 879 278 beschriebene Substanz, welche folgende Formel besitzt.

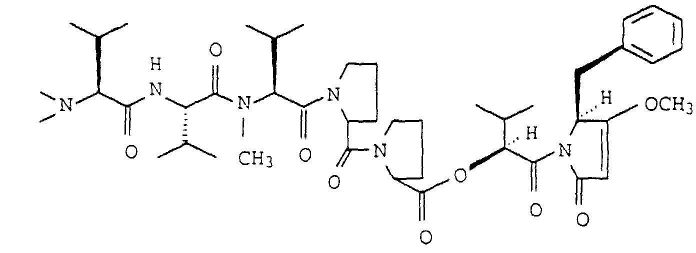

Dolastatin 15 findet wegen seiner hohen Wirksamkeit gegen verschiedene Tumoren großes Interesse. Seine Isolierung aus dem schwierig zugänglichen Seehasen ist langwierig und zeitaufwendig, und das Verfahren liefert den Wirkstoff in mäßiger Ausbeute und in wenig reproduzierbarer Qualität. Um den Wirkstoff in Gramm-Mengen für Tierversuche verfügbar zu machen, wurde von Pettit et al. (J. Am. Chem. Soc. 113 [1991] 6692-6693) eine Synthese-methode entwickelt. Zentrales Zwischenprodukt ist dabei das Tetrapeptid der Formel Ia

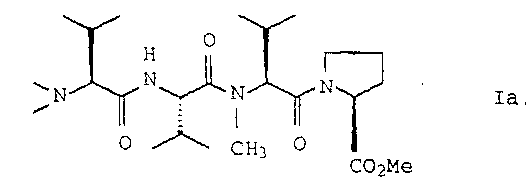

In WO 93/23424 werden antineoplastische Wirkstoffe beschrieben, die in ihrer Wirkung Dolastatin 15 übertreffen. Zahlreiche Wirkstoffe von WO 93/23424 lassen sich aus Tetrapeptiden der Formel I 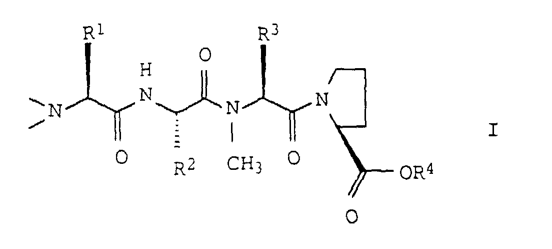 herstellen, worin R¹-R⁴ C₁-C₆-Alkylgruppen darstellen.

Um für klinische Prüfungen ausreichende Substanzmengen der Peptide von WO 93/23424 und von Dolastatin 15 verfügbar zu machen, mußte ein technisch realisierbares Verfahren für die Herstellung der Tetrapeptide I gefunden werden.

Die in WO 93/23424 beschriebene Festphasensynthese ist für die Herstellung größerer Produktmengen ungeeignet. Sie liefert verunreinigtes Produkt, das mühsam chromatographisch aufgereinigt werden muß.

Pettit et al. (J. Am. Chem. Soc. 113 [1991] 6692-6693) beschreiben eine elegante Laborsynthese für Ia. Dabei wird das Tripeptid Val―MeVal―Pro―OMe mit 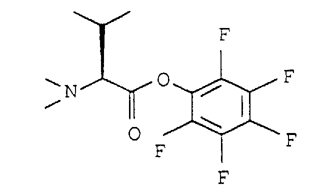 (Kurzbezeichnung: Me₂Val―OPfp) zum Tetrapeptid Ia umgesetzt.

Die Synthese von Me₂Val―OPfp erfolgt nach Pettit et al. (US-PS 49 78 744) wie folgt:

Zunächst wird Valin an Stickstoff zweifach methyliert. Das so erhaltene Dimethylvalin muß mit einem Kondensationsmittel und Pentafluorphenol (HO-Pfp) umgesetzt werden.

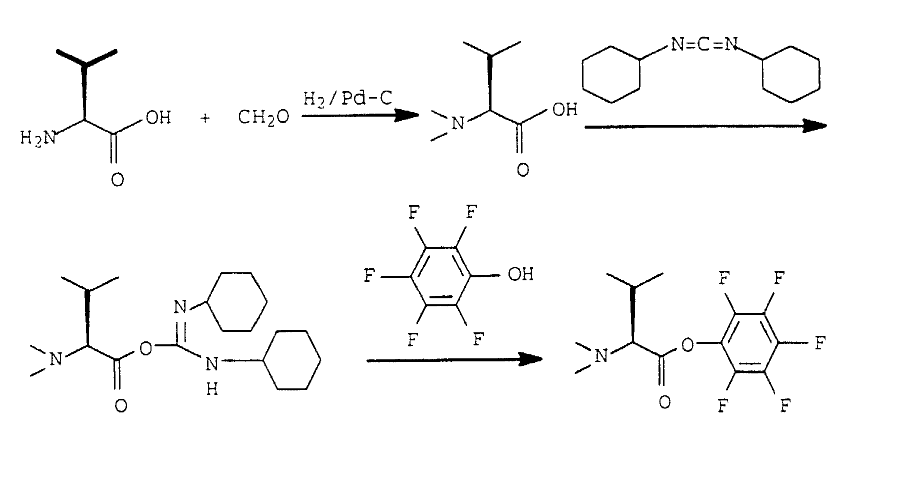

Dieses Verfahren ist durch die Vielzahl der erforderlichen Reaktionsschritte sehr umständlich. Hinzu kommt, daß Pentafluorphenol ein sehr teures Reagenz ist, das für technische Synthesen nicht in genügender Menge zur Verfügung steht. Weiter führt seine Verwendung zu fluorhaltigen Abfällen, die nur schwer und möglicherweise unter Dioxinbildung zu entsorgen sind.

Die Verwendung von Kondensationsmitteln bei der Synthese führt ebenfalls zur Problemen. So ist es bekannt, daß der Umgang mit Dicyclohexylcarbodiimid zur Sensibilisierung und schwersten allergischen Reaktionen führen kann. Bei der Aufarbeitung der Ansätze fällt der entsprechende Harnstoff an, der nur umständlich und oft nur unvollständig aus dem Produkt entfernt werden kann. Carbodiimide, die wie N-Ethyl-N'-dimethylaminopropylcarbodiimid zu wasserlöslichen Harnstoffen abreagieren, sind extrem teuer und stehen nicht in technischen Mengen zur Verfügung.

Auch der Ersatz von Pentafluorphenol/Carbodiimid durch Pentafluorphenyltrifluoracetat oder dessen Carbonate, z.B. Pentafluorphenyl-1,2,2,2-tetrachlorethylcarbonat (J. Org. Chem. 52 [1987] 2364), bringt keine Vorteile.

Bei der Aktivester-Bildung und der Peptidkupplung tritt eine Racemisierung auf, die bei dieser Methode ein großes Problem darstellt (s. J. Jones: The Chemical Synthesis of Peptides, Oxford 1991, S. 57).

Eine andere publizierte Methode zur Verknüpfung von Dimethylvalin mit anderen Aminosäuren (T. Shioiri et al.: Tetrahedron 49 [1993] 1913-1924), die Diethylphosphorylcyanid (DEPC) als Kupplungsreagenz verwendet, bringt ebenfalls schwerwiegende Nachteile mit sich: DEPC ist sehr teuer, nicht in großen Menge erhältlich, ätzend und sehr giftig. Die cyanidhaltigen Mutterlaugen und Waschflüssigkeiten müssen als Sondermüll entsorgt werden.

Die Erfindung betrifft nun ein Verfahren zur Herstellung der Tetrapeptide I, das mit Zwischenprodukten auskommt, die in großen Mengen verfügbar sind, das racemisierungsfrei und ohne Gefährdung der Umwelt durchführbar ist.

Gegenstand der Erfindung ist ein Verfahren zur Herstellung von Verbindungen der Formel 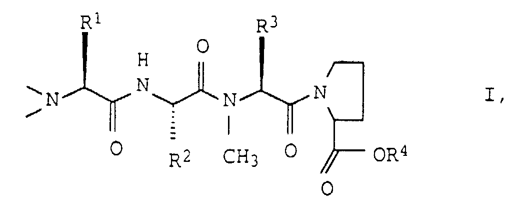 worin R¹, R², R³ und R⁴ gleich oder verschieden sind und C₁₋₆-Alkylgruppen darstellen, dadurch gekennzeichnet, daß man eine Verbindung der Formel II 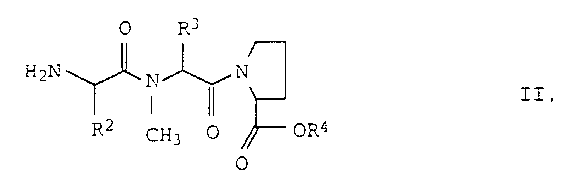 worin R², R³ und R⁴ die oben angegebene Bedeutung haben, mit einer Aminosäure der Formel III

R¹―CH(NHZ)―COOH III,

worin R¹ die oben angegebene Bedeutung besitzt und Z eine Benzyloxycarbonylschutzgruppe darstellt, die am Phenylring substituiert sein kann, kondensiert und die erhaltene Verbindung an der NH₂-Gruppe zweifach methyliert.

In der Formel I sind R¹, R² und R³, die gleich oder verschieden sind, vorzugsweise Ethyl-, n-Propyl-, Isopropyl-, t-Butyl-, sec-Butyl-, 2-Methyl-n-but-1-yl- oder 3-Methyl-n-but-1-yl-Gruppen. R⁴ ist vorzugsweise eine Methyl- oder Ethylgruppe.

Die Aminogruppen in dem Tetrapeptid besitzen vorzugsweise die L-Konfiguration.

Die Kupplungsreaktion von II mit III kann beispielsweise nach der "Gemischte-Anhydrid"-Methode durchgeführt werden (siehe Houben-Weyl, Bd. XV/2, 1974; J. Am. Chem. Soc. 74 [1952] 676; Coll. Czechoslov. Chem. Comm. 27 [1962] 1273). Die als Kupplungsreagenzien verwendeten Carbonsäurechloride (vorzugsweise Pivalinsäurechlorid, 2-Ethylbuttersäurechlorid, Isovaleriansäurechlorid) bzw. Chlorameisensäureescer (bevorzugt werden der Methyl-, Ethyl-, Isopropyl-, Isobutyl-, Phenyl-, Chlorethyl- und Trichlormethylester eingesetzt) sind günstige, in technischen Mengen erhältliche Reagenzien, die sich auch für Synthesen im technischen Maßstab gut eignen. Besonders vorteilhaft ist die Tatsache, daß die erfindungsgemäßen Kupplungen ohne Racemisierung ablaufen.

Als Lösungsmittel eignen sich Tetrahydrofuran, Dioxan, Acetonitril, Dimethylsulfoxid, Essigsäureethylester, Dimethylformamid, Methylenchlorid, Toluol, N-Methylpyrrolidon und ihre Mischungen. Bevorzugt sind Methylenchlorid, Toluol bzw. ihre Gemische. Als Basen eignen sich für die Umsetzung besonders: Triethylamin, Tributylamin, N-Ethylpiperidin, Diisopropylethylamin und N-Methylmorpholin; bevorzugt sind Triethylamin und N-Methylmorpholin.

Die Reaktion wird bei Temperaturen zwischen -40°C und +30°C ausgeführt, bevorzugt ist der Temperaturbereich -15°C bis +15°C.

Gegenstand der Erfindung sind weiter die Verbindungen der Formel I, worin jedoch R¹, R² und R³ nicht gleichzeitig Isopropylreste darstellen, wenn R⁴ eine Methylgruppe bedeutet, sowie deren Salze mit verschiedenen Säuren.

Als Säuren seien beispielsweise genannt: Salzsäure, Citronensäure, Weinsäure, Milchsäure, Phosphorsäure, Methansulfonsäure, Essigsäure, Ameisensäure, Maleinsäure, Fumarsäure, Malonsäure, Bernsteinsäure, Äpfelsäure, Schwefelsäure, Benzoesäure und Oxalsäure.

Die Verbindungen der Formel I eignen sich sehr gut zur Herstellung von Dolastatin 15 und zahlreicher Verbindungen, die in WO 93/23424 (vgl. Beispiele 214-246 und andere) beschrieben sind und die sich durch eine hohe antineoplastische Wirksamkeit auszeichnen.

Die Verbindungen der Formel I sind ebenfalls wirksam gegen feste Tumoren (Tumoren der Lunge, der Brust, des Darms, der Blase, des Enddarms, der Gebärmutter, der Prostata), gegen Leukämie, Lymphome und andere neoplastische Erkrankungen.

Zur Abkürzung der Aminosäure wird der übliche Dreibuchstabencode benutzt. Me₂Val bedeutet N,N-Dimethyl-L-Valin, MeVal N-Methyl-Valin, Me Methyl, tert. Leu tertiär-Leucin (HOOC―CH(NH₂)C(CH₃)₃), Me₂ tert. Leu N,N-Dimethyl-tertiär-Leucin und Me tert. Bu Ala N-Methyl-tertiär-Butyl-Alanin (HOOC―CH(NHCH₃)―CH₂―C(CH₃)₃), Hyp 4-Hydroxyprolin, Bu n-Butyl, tBu tertiär-Butyl, Hx n-Hexyl und Et Ethyl.

### Beispiele

### Beispiel 1

### Herstellung von Z-Val-Val-MeVal-Pro-OMe

In einem 400-1-Kessel wurden 17,84 kg (70,88 mol) Z-Valin und 4,59 kg (74,42 mol) Triethylamin in 170 1 Methylenchlorid gelöst. Zu dieser Lösung dosierte man bei -5 bis -10°C 8,58 kg (70,88 mol) Pivalinsäurechlorid zu. Nach einer Reaktionszeit von 2 h bei -5°C ließ man bei -5°C eine Lösung von 24,2 kg Val-MeVal-Pro-OMe in 86 1 Methylenchlorid zulaufen. Nach weiteren 2 h bei -5°C wurde auf 20°C erwärmt und bei dieser Temperatur 12 h gerührt. Zur Aufarbeitung wurden 50 1 Wasser zugegeben. Nach Abtrennung der wäßrigen Phase wurde die organische Phase einmal mit 40 1 2 N Salzsäure und zweimal mit je 40 1 2 N Natronlauge extrahiert. Nach Neutralwaschen der organischen Phase wurde das Lösungsmittel Methylenchlorid abdestilliert und durch 300 1 Diisopropylether ersetzt. Die Emulsion des Produktöls wurde zur Kristallisation des Produkts auf 60°C erwärmt, mit Impfkristallen versetzt und 7 h bei 60°C gehalten. Zur Vervollständigung der Kristallisation wurde nacheinander 5 h bei 50°C und 5 h bei 40°C weitergerührt und anschließend auf 20°C abgekühlt. Die Kristallsuspension wurde über einen 120-1-Druckfilter abgelassen.

| | |
|---|---|
| Ausbeute | 32,2 kg ≙ 79 % d.Th. |
| Gehalt | 98,5 % (HPLC-Flächenprozent) |
| Schmelzpunkt | 134 - 135°C |

### Beispiel 2

### Herstellung von Me₂Val-Val-MeVal-Pro-OMe x HCl

In einem 400-1-Hydrierkessel wurden 20 kg (34,8 mol) Z-Val-Val-MeVal-Pro-OMe zusammen mit 2 kg 5 %iger Palladiumkohle in 200 1 Methanol vorgelegt. Anschließend wurde unter Kühlung bei 20°C so lange Wasserstoff eingeleitet, bis in der Reaktionslösung kein Edukt mehr nachzuweisen war. Anschließend fügte man 8,46 kg 37 %ige (104 mol) Formalin-Lösung zu und hydrierte bis zur Beendigung der Wasserstoffaufnahme bei 20°C weiter. Der Katalysator wurde beim Ablassen des Kesselinhalts abfiltriert. Zur Aufarbeitung wurde in einem 400-1-Email-Kessel im Wasserstrahl-Vakuum bis auf 50 1 aufkonzentriert. Danach wurden 200 1 Isopropanol zugegeben und erneut auf 50 1 eingeengt. Anschließend löste man in 135 1 Methyl-tert.-butylether und fügte unter Kühlung bei 20°C ein Äquivalent isopropanolische HCl zu. Die entstandene Suspension wurde noch 3-4 h bei 20°C und 2 h bei 0-5°C weitergerührt und dann über einen 120-1-Druckfilter ausgetragen. Der Filterkuchen wurde einmal mit 50 1 frischem Methyl-tert.-butylether gewaschen.

| | |
|---|---|
| Ausbeute | 16,2 kg ≙ 92,3 % d.Th. |
| Gehalt | 99,9 % (HPLC-Flächenprozent) |
| Schmelzpunkt | 224°C (Zersetzung) |

### Beispiel 3

Die Zwischenstufe Val-Val-MeVal-Pro-OMe war ebenfalls isolierbar, wenn nach der ersten Hydrierstufe wie folgt aufgearbeitet wurde:

Die Reaktionslösung wurde vom Katalysator abgetrennt und eingeengt. Der Rückstand wurde in Essigester aufgenommen. Die Essigesterlösung wird zweimal mit 2 N Salzsäure extrahiert. Die saure wäßrige Phase wurde mit Natronlauge auf pH 9 gestellt und zweimal mit Methylenchlorid extrahiert. Die Methylenchloridphase wurde anschließend neutral gewaschen und eingedampft.
HPLC 96,8 %
1H-NMR (400 MHz, CDCl3 / TMSint.):
δ (ppm): 0,84 - 1,08 (m, 18H); 1,45 - 1,6 (s, breit, NH2); 1,85 - 2,15 (m, 4H); 2,18 - 2,38 (m, 3H); 3,15 (s, N-CH3); 3,25 (d, 1H); 3,65 - 3,75 (m, 1H); 3,73 (s, O-CH3); 3,9 - 4,05 (m, 1H); 4,38 - 4,45 (m, 1H); 4,73 - 4,83 (m, 1H); 5,12 (d, 1H); 7,9 (d, NH)

### Beispiel 4

Die Herstellung von Me₂Val-Val-MeVal-Pro-OMe x HCl gelang auch nach der folgenden Herstellvorschrift, bei der auf eine Isolierung und Reinigung der Zwischenstufe Z-Val-Val-MeVal-Pro-OMe verzichtet wurde:

In einem 4-1-Kolben wurden 128 g (0,51 mol) Z-Valin (Gehalt 99,8%) und 55,1 g (0,54 mol) Triethylamin (Gehalt: 99 %) in 1,2 1 Methylenchlorid gelöst. Zu dieser Lösung dosierte man bei -5°C bis -10°C 62,1 g (0,51 mol) Pivalinsäurechlorid (Gehalt: 99 %). Nach einer Reaktionszeit von 2 h bei -5°C ließ man eine Lösung von 174,6 g (0,51 mol) Val-MeVal-Pro-OMe in 0,8 1 Methylenchlorid zulaufen, rührte weitere 2 h bei -5°C und dann unter Erwärmung auf 20°C weitere 12 h. Danach wurde der Ansatz mit 370 ml Wasser versetzt. Nach der Phasentrennung wurde die Methylenchloridphase einmal mit 290 ml 2 N Salzsäure, zweimal mit je 290 ml 2 N Natronlauge und dreimal mit 370 ml Wasser gewaschen. Anschließend wurde das Methylenchlorid abgedampft und durch 3 1 Methanol ersetzt. Zu dieser Lösung gab man eine Aufschlämmung von 30 g 5 %iger Palladiumkohle in 110 ml Wasser und hydrierte bei 25°C mittels Begasungsrührer und Wasserstoffbürette, bis ein Äquivalent Wasserstoff aufgenommen war. Danach gab man 123 g (1,53 mol) 37 %ige Formalinlösung zu und hydrierte bis zur Aufnahme weiterer 2 Äquivalente Wasserstoff. Danach wurde vom Katalysator abgetrennt und am Rotationsverdampfer eingedampft. Das verbleibende Öl wurde in 670 ml Isopropanol und 2,6 1 Methyl-tert.-butylether gelöst. Zu dieser Lösung gab man ein Äquivalent isopropanolische HCl. Die entstandene Suspension wurde bei 20°C 12 h weitergerührt und dann abgesaugt. Der Filterkuchen wurde mit wenig frischem Methyl-tert.-butylether gewaschen und anschließend im Vakuum bei 40°C getrocknet.

| | |
|---|---|
| Ausbeute | 182,8 g ≙ 71 % d.Th. |
| Gehalt | 99,4 % (HPLC-Flächenprozent) |
| Schmelzpunkt | 224°C (Zersetzung) |

Analog Beispiel 1 bis 4 lassen sich herstellen:

| | |
|---|---|
| 5 | Me₂Val - Val - MeVal - Pro - OEt |
| 6 | Me₂Ile - Ile - MeVal - Pro - OMe |
| 7 | Me₂Val - tert.Leu - MeVal - Pro - OtBu |
| 8 | Me₂Val - Leu - MeVal - Pro - OMe |
| 9 | Me₂Leu - Val - MeVal - Pro - OEt |
| 10 | Me₂tert.Leu - Val - MeVal - Pro - OBu |
| 11 | Me₂Val - tert.Leu - MeVal - Pro - OtBu |
| 12 | Me₂tert.Leu - tert.Leu - MeVal - Pro - OHx |
| 13 | Me₂Leu - Val - MeVal - Pro - OEt |
| 14 | Me₂Val - Val - Meter.Leu - Pro - OEt |
| 15 | Me₂Val - Val - Metert.BuAla - Pro - OHx |
| 16 | Me₂Val - tert.Leu - MeVal - Pro - OMe |
| 17 | Me₂Val - Ile - MeVal - Pro - OEt |
| 18 | Me₂tert.Leu - Val - Metert.Leu - Pro - OtBu |

### Verwendungsbeispiele

### Herstellung von Me₂Val-Val-MeVal-Pro-Pro-NHBzl x HCl

In einem 400-1-Kessel wurden 15,9 kg (31,5 mol) Me₂-Val-Val-MeVal-Pro-OMe x HCl (Gehalt: 99,5 %) zusammen mit 140 1 Toluol und 15 1 Methanol vorgelegt. Dazu gab man 3,15 kg (76,38 mol) Natriumhydroxid-Pellets. Nach vollständiger Verseifung, d.h. nach etwa 3 h bei 20°C, wurde durch Zugabe von isopropanolischer HCl neutralisiert. Anschließend wurde im Azeotrop mit Toluol bei 100 mbar bis zur Alkohol- und Wasserfreiheit destilliert. Das abdestillierte Lösungsmittelvolumen wurde sukzessive durch Toluol ersetzt. Anschließend wurden 80 1 Methylenchlorid und 6,44 kg (63,0 mol) Triethylamin zugegeben, auf -5°C abgekühlt und bei dieser Temperatur 3,84 kg (31,5 mol) Pivalinsäurechlorid zudosiert. Nach 2 h Reaktionszeit gab man bei -5°C bis 0°C 7,6 kg (31,5 mol) Pro-NHBzl x HCl portionsweise zu. Nach 2 h bei -5°C erwärmte man auf 20°C und ließ noch weitere 6 h reagieren. Anschließend destillierte man bei 500 mbar das zugesetzte Methylenchlorid ab und fügte 80 1 Toluol hinzu. Danach gab man 50 1 Wasser zu und stellte den pH-Wert der wäßrigen Phase auf pH 9 ein. Nach kräftigem Rühren wurde die wäßrige Phase abgetrennt und die organische Phase einmal mit 25 1 Wasser nachgewaschen. Anschlie-ßend wurde die organische Phase zweimal mit je 50 1 2N Salzsäure extrahiert. Das Produkt wurde aus der sauren Wasserphase nach Einstellung des pH auf 9 durch 3faches Extrahieren mit je 50 1 Methylenchlorid zurückextrahiert. Nach Neutralwaschen der Methylenchloridphase wurde das Methylenchlorid abdestilliert und durch 180 1 Methylethylketon ersetzt. Die Lösung wurde auf 40°C erwärmt und mit einem Äquivalent (31,5 mol) isopropanolischer HCl versetzt. Die entstandene Suspension wurde auf 60°C erwärmt und anschließend 12 h gerührt. Anschließend wurde auf 20°C abgekühlt und weitere 5 h gerührt. Danach wurde auf 5°C abgekühlt und über einen 120-1-Druckfilter abgelassen. Der Filterkuchen wurde mit 60 1 frischem, 5°C kaltem, Methylethylketon gewaschen. Nach Vortrocknung auf dem Filter wurde das Produkt im Vakuum bei 40°C bis zur Gewichtskonstanz getrocknet.

| | |
|---|---|
| Ausbeute | 14,36 kg ≙ 67 % d.Th. |
| Gehalt | 99,6 % (HPLC-Flächenprozent) |
| Schmelzpunkt | 214°C (Zersetzung) |

Analog dem Verwendungsbeispiel lassen sich herstellen:

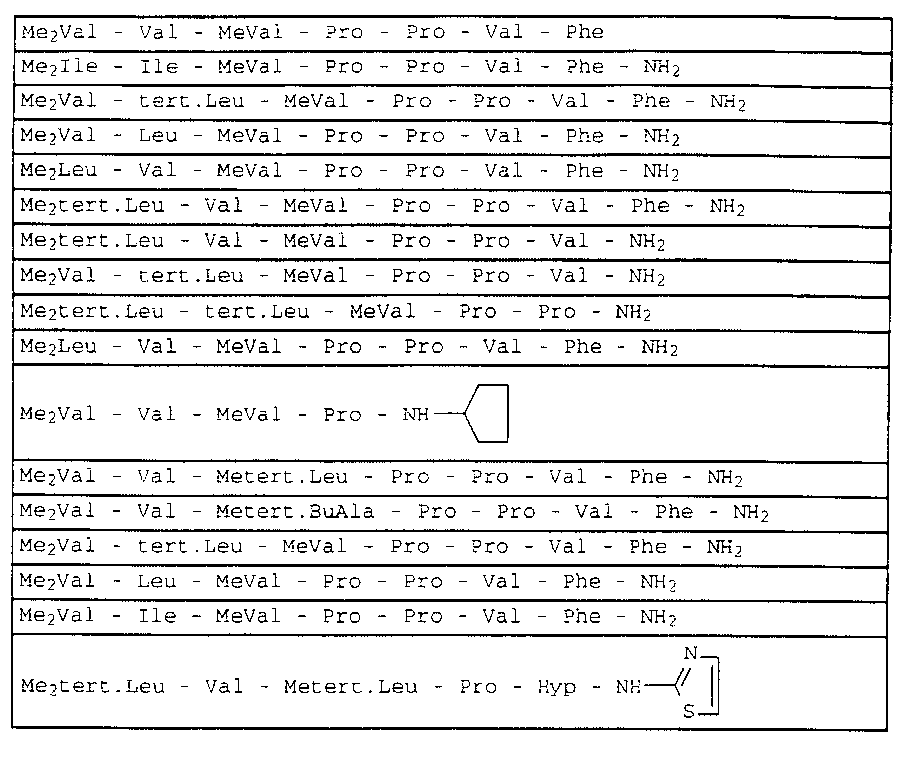

## Patentansprüche

1. Verfahren zur Herstellung von Verbindungen der Formel 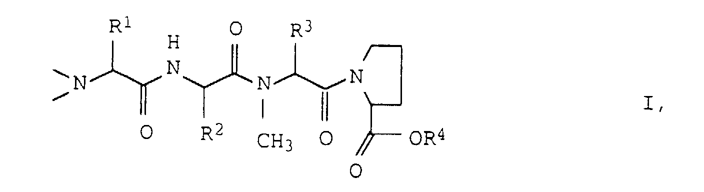 worin R¹, R², R³ und R⁴ gleich oder verschieden sind und C₁₋₆-Alkylgruppen darstellen, dadurch gekennzeichnet, daß man eine Verbindung der Formel II 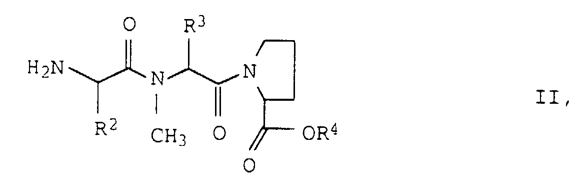 worin R², R³ und R⁴ die oben angegebene Bedeutung haben, mit einer Aminosäure der Formel III
R¹―CH(NHZ)―COOH III,
worin R¹ die oben angegebene Bedeutung besitzt und Z eine Benzyloxycarbonylschutzgruppe darstellt, die am Phenylring substituiert sein kann, kondensiert, die Schutzgruppe entfernt und die erhaltene Verbindung an der NH₂-Gruppe zweifach methyliert.

2. Verbindungen der Formel I gemäß Anspruch 1 in Form von Salzen mit Säuren.

3. Verbindungen der Formel I gemäß Anspruch 1, worin jedoch die Reste R¹, R² und R³ nicht gleichzeitig Isopropylreste darstellen, wenn R⁴ eine Methylgruppe bedeutet, sowie deren Salze mit Säuren.

## Claims

1. A process for preparing compounds of the formula 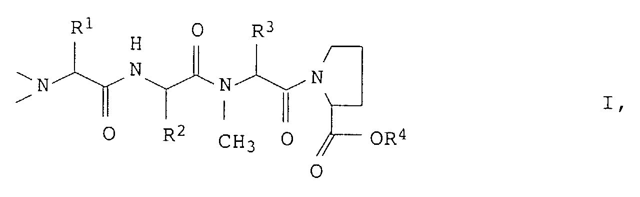 where R¹, R², R³ and R⁴ are identical or different and are each C₁₋₆-alkyl, which comprises condensing a compound of the formula II 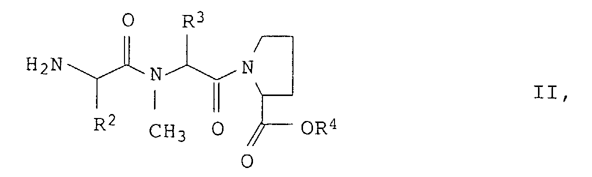 where R², R³ and R⁴ have the abovementioned meaning, with an amino acid of the formula III
R¹―CH(NHZ)―COOH III,
where R¹ has the abovementioned meaning and Z is a benzyloxycarbonyl protective group which may be substituted on the phenyl ring, removing the protective group and dimethylating the resulting compound on the NH₂ group.

2. A compound of the formula I as defined in claim 1 in the form of salts with acids.

3. A compound of the formula I as defined in claim 1, but in which the R¹, R² and R³ radicals are not all isopropyl radicals when R⁴ is methyl, and the salts thereof with acids.

## Revendications

1. Procédé de préparation de composés de formule 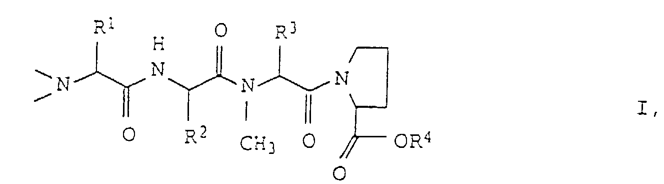 dans laquelle R¹, R², R³ et R⁴, ayant des significations identiques ou différentes, représentent chacun un groupe alkyle en C1-C6, caractérisé par le fait que l'on condense un composé de formule II 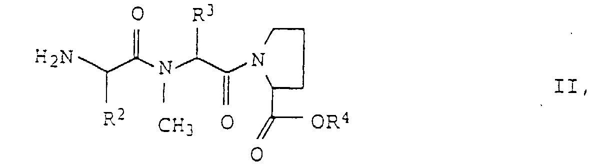 dans laquelle R², R³ et R⁴ ont les significations indiquées ci-dessus, avec un amino-acide de formule III
R¹―CH(NHZ)―OOH III,
dans laquelle R¹ a les significations indiquées ci-dessus et Z représente un groupe protecteur benzyloxycarbonyle qui peut être substitué sur le cycle phényle, on élimine le groupe protecteur et on soumet le composé obtenu à double méthylation sur le groupe NH₂.

2. Composés de formule I selon la revendication 1 à l'état de sels d'acides.

3. Composés de formule I selon la revendication 1, pour lesquels les symboles R¹, R² et R³ ne peuvent représenter tous des groupes isopropyle lorsque R⁴ représente un groupe méthyle, et leurs sels d'acides.
